# EUROPEAN PATENT APPLICATION

(11) **EP 4 332 574 A1**
(43) Date of publication of application: **06.03.2024**
(21) Application number: 22192620.7
(22) Date of filing: 29.08.2022
(51) Int. Cl.: G01N 33/52, G01N 33/574

(54) **SELF-ASSEMBLED CHEMOSENSORS FOR DETECTING POLYAMINES IN PHYSIOLOGICAL MEDIA AND BIOFLUIDS**

(71) Applicant: Karlsruher Institut für Technologie, 76131 Karlsruhe (DE)
(72) Inventor: Prabodh, Amrutha, 76133 Karlsruhe (DE); Grimm, Laura, 76761 Rülzheim (DE); Biedermann, Frank, 76137 Karlsruhe (DE)
(74) Representative: Gille Hrabal Partnerschaftsgesellschaft mbB Patentanwälte

(57) **Abstract**

The present inventions relate to chemosensors comprising a pillar[*n*]arene-based host molecules and a dicationic dyes and the use thereof in analytical methods for determining polyamines, such as in particular biogenic polyamines. The invention further relates to methods for preparing such chemosensors and to methods for detecting, determining, or analysing polyamines with fluorescence spectroscopy by using the chemosensors.

## Description

### INTRODUCTION

The present invention relates to chemosensors comprising a pillar[n]arene-based host molecule and a dicationic dye and the use thereof in analytical methods for determining polyamines, particularly biogenic polyamines. The invention further relates to methods for preparing such chemosensors and to methods for detecting, determining, or analysing polyamines with fluorescence spectroscopy by using the chemosensors.

### BACKGROUND OF THE INVENTION

Polyamines play a major role in the human body, as they are involved in several biological mechanisms, including cell growth and proliferation. At optimum levels, polyamines reduce the risk of many cardiovascular diseases and exhibit anti-aging properties. Dietary spermidine supplements can contribute to an increased life span in humans and protect them from cardiac aging and age-induced memory impairment. At the same time, altered polyamine levels in the body are often associated with several diseases, including Alzheimer's and Parkinson's disease, strokes, heart failure, and cancer *(*Science, 2018, 359, eaan2788).

For instance, elevated levels of polyamines are found in the urine and blood serum of cancer patients and thus, serve as useful diagnostic markers for early-stage disease detection. Hence, it is essential to regularly monitor and maintain the level of polyamines in the body for proper cell functioning and to control the course of a disease to a greater extent.

Current methods for polyamine detection in diagnostic laboratories include separation techniques such as high-performance liquid chromatography (HPLC), gas chromatography (GC), and capillary electrophoresis. These techniques are very precise, however, they are time-consuming, complex, and expensive. Furthermore, due to the required high-tech instrumentation in combination with skilled technicians, polyamine detection is often not financially viable for small laboratories. In addition, HPLC assay methods for the detection of polyamines, in particular for the detection and quantification in biofluids, involve laborious sample pre-treatment and relatively long assay times.

Therefore, fast and broad analysis of a large number of samples for routine screening of polyamines in biofluids is still impossible though it could improve many areas such as the early detection and personalized treatment of diseases. Further, home applications or rapid tests in doctors' offices are not yet possible. The development of rapid, cost-efficient, and widely applicable methods for polyamine detection, including large-scale sample screening techniques and methods for point-of-care use, could revolutionize polyamine sensing and aid in detecting early-stage diseases. In addition, patients' health status monitoring would be improved, and strategies for personalized medicine in medical settings would become better available. Hence, a fast and broad analysis of biofluid samples for routine polyamine screening and easy-to-use test kits for point-of-care applications are needed.

The inventors of the present invention developed reliable analytical approaches for detecting polyamines in aqueous saline buffers and biofluids such as blood, urine, and saliva. These polyamines function as health indicators in the human body.

### PRIOR ART

Fluorescence-based sensing assays are highly desirable analytical methods for the detection of polyamines due to their technical simplicity, low costs, and the possibility of real-time detection of analytes with high sensitivity and high selectivity. Further, fluorescence-based sensing assays can be used in high-throughput screenings (HTS) to achieve a large-scale analysis of samples. Therefore, fluorescence-based sensing assays offer rapid, cost-efficient, and widely applicable methods for analysing biofluids.

In the past few years, the number of reported fluorescent chemosensors and nanoparticle-based probes for the purpose of polyamine detection has increased. Unfortunately, the reported systems suffer from various limitations affecting their practical utility for sensing applications in real biofluid samples. Examples include the use of a negatively charged micelle for the charge-mediated recognition of polycationic polyamines resulting in a fluorescence "turn-off' signal (Chemical Communications, 2012, 48, 5841) or an analyte-directed formation of emissive excimers of a sulfonated probe in the presence of polyamines (Chemical Communications, 2016, 52, 10648) or using fluorescence-mediated host-guest recognition interactions (Supramolecular Chemistry, 2016, 28, 499). The functionality of such previously reported fluorescent chemosensors was evaluated only in deionized water or low-salt buffers and not assessed in real biofluids. As nanoparticle-based probes, functionalized gold nanoparticles were reported for polyamine detection through electrostatic interactions of the negatively charged nanoparticles with the polyamines (Chemistry-A European Journal, 2011, 17, 11978). Recently, a reactive probe based on an agarose-coumarin hydrogel was introduced for the detection of spermine and spermidine in aqueous buffers and biofluids spiked with unphysiological high concentrations of polyamines (ACS Applied Bio Materials, 2019, 2, 2374).

The previously reported fluorescent chemosensors are only operational in aqueous media and low-salt buffers. The lack of stability in saline and biorelevant media such as human urine or saliva, containing high millimolar salt concentrations, significantly limits their applicability as their functionality in biofluids is inhibited. Additionally, inferior fluorescence "turn-off' signals shown in the sensing assays of previously reported fluorescent chemosensors and the long response times of reactive chemical probes are disadvantageous and make it challenging to regularly monitor variations in polyamine levels in biofluids in relation to the change in fluorescence signal of the chemosensor. Furthermore, a high sensitivity and selectivity of the chemosensor towards the analyte of interest are critical factors for its performance in real-world environments. The previously reported fluorescent chemosensors exhibit a lack of sensitivity and selectivity in biofluids for the polyamines of interest and suffer from strong interferences with other biogenic amines, metal ions, and naturally occurring small biomolecules in the media. This results in strong matrix influences, which hampers polyamine detection and distinction in biofluids.

### OBJECT OF THE INVENTION

The object of the present invention was to provide new possibilities for detecting and determining polyamines, in particular biogenic polyamines, which lack the disadvantages of so far available methods. A particular object of the invention was to provide a new assay-based method for the qualitative and/or quantitative determination of polyamines, in particular biogenic polyamines. A further object of the present invention was to provide new methods for detecting and determining polyamines, in particular biogenic polyamines, which are applicable in saline or complex physiological reaction media. A further object of the present invention was to provide a new method for detecting and determining polyamines, in particular biogenic polyamines, using fluorescence-based methods. A further object of the invention was to develop new and improved chemosensors which are suitable for detecting and determining polyamines, in particular biogenic polyamines, in particular under the aforementioned determination conditions.

These objects were solved by the present invention, which provides reliable analytical approaches for detecting polyamines, in particular biogenic polyamines, which function as health indicators in the human body. The novel approaches designed by the inventors are further particularly suitable for the determination of polyamines in aqueous saline buffers and biofluids such as blood, urine, and saliva. The inventors of the present invention found that chemosensors comprising pillar[n]arene-based host molecules, acting as molecular containers, and dicationic dyes result in novel and improved artificial chemosensors that provide an easy and fast spectroscopic signal response towards the presence of polyamines, show distinguishable fluorescence "turn-on" signals for polyamines, and enable a simple, fast, and low-cost method for real-time analyte detection with significant sensitivity. The invention, therefore, offers new possibilities for application in the field of analytical chemistry and physiological analysis, including medical diagnostics, early disease detection, personalized medicine strategies, life science research, and instrumental analysis.

The present invention and its embodiments and aspects are described in the claims and in more detail below.

### SUMMARY OF THE INVENTION

The present invention relates to new chemosensors comprising of a pillar[n]arene-based host molecule and a dicationic dye and their use in analytical methods for determining polyamines, such as in particular biogenic polyamines. The present invention includes the following aspects particularly:
[1] A chemosensor comprising of a pillar[n]arene-based host molecule and a dicationic dye.
[2] The chemosensor according to [1], which is an optically active chemosensor.
[3] The chemosensor according to [2], wherein the optically active chemosensor contains a fluorescent dicationic dye.
[4] The chemosensor according to any one of [1] to [3], wherein the host molecule is a sulfated pillar[n]arene.
[5] The chemosensor according to any one of [1] to [4], wherein the host molecule is a sulfated pillar[n]arene (P5AS) having the structure 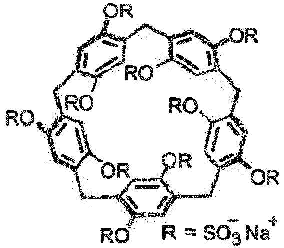
[6] The chemosensor according to any one of [1] to [5], wherein the dicationic dye has at least two quaternary ammonium groups.
[7] The chemosensor according to any one of [1] to [6], wherein the dicationic dye has a diazapyrenium core.
[8] The chemosensor according to any one of [1] to [7], wherein the dicationic dye is selected from MDAP and BrDAP according to the following structures 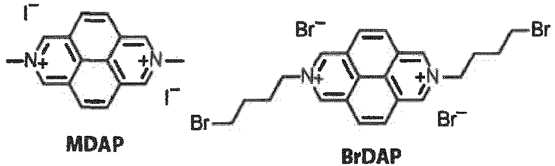
[9] The use of the chemosensors according to any one of [1] to [8] for detecting and determining polyamines, in particular biogenic polyamines.
[10] The use according to [9], wherein the polyamines are polycationic polyamines.
[11] The use according to [9] or [10], wherein the polyamines are selected from the group comprising spermine, spermidine, and cadaverine.
[12] The use according to any one of [9] to [11] for detecting and determining biogenic polyamines in a fluorescence-based assay.
[13] The use according to any one of [9] to [12] for detecting and determining biogenic polyamines in physiological media or endogenous fluids or secretions, preferably in PBS, urine, saliva, blood, sweat, semen, amniotic fluid, tear fluid, or cerebrospinal fluid (CSF).
[14] A method for preparing the chemosensors according to any one of [1] to [8] by mixing an aqueous solution containing the host molecules as defined in [1] to [5] with an aqueous solution containing the dicationic dye as defined in [1] or [6] to [8] and shaking the mixture to obtain a self-assembled host-dye chemosensor.
[15] A method for detecting, determining, or analysing polyamines with fluorescence spectroscopy by using the chemosensors according to any one of [1] to [8].
[16] The method according to [15], comprises the steps:
   i) preparing or providing the chemosensors according to any of [1] to [8];
   ii) providing the chemosensors of step i) in an aqueous solution;
   iii) providing an aqueous test sample;
   iv) contacting the chemosensors of step i) or ii) with the test sample iii);
   v) determination of the polyamines by fluorescence spectroscopy.
[17] The method according to [15] or [16], comprises an additional step of preparing a layer
   (a) of the aqueous solution according to ii), or
   (b) of a mixture of the chemosensors according to i) and the aqueous test sample according to iii), or
   (c) of a mixture of the aqueous solution according to ii) and the aqueous test sample according to iii)
   by means of a spraying or aerosol printing method on a support material and determination of the polyamines in such a layer by fluorescence spectroscopy.
[18] The method according to any one of [15] to [17], wherein the polyamines are polycationic polyamines.
[19] The method according to any one of [15] to [18], wherein the polycationic polyamines are biogenic polyamines, preferably selected from the group comprising spermine, spermidine, and cadaverine.
[20] The method according to any one of [15] to [19], wherein the test sample of step iii) is a physiological medium such as PBS, an endogenous fluid or secretion sample obtained from a human or an animal, such as urine, saliva, blood, sweat, semen, amniotic fluid, tear fluid, or cerebrospinal fluid.
[21] The method according to any one of [15] to [20] for detecting, determining, or analysing spermine, wherein the chemosensor comprises BrDAP as a dicationic diazapyrenium dye.
[22] The use of MDAP and BrDAP as fluorescence dyes in an assay for detecting and determining polyamines, in particular biogenic polyamines, more particularly polycationic polyamines, such as preferably spermine, spermidine, and cadaverine.
[23] A diagnostic kit comprising the chemosensor according to any one of [1] to [8].
[24] The use of the diagnostic kit according to [23] for the analysis of polyamines, in particular biogenic polyamines, more particularly polycationic polyamines, such as preferably spermine, spermidine, and cadaverine.

### DETAILED DESCRIPTION OF THE INVENTION

The inventors of the present invention found that newly developed chemosensors comprising pillar[n]arene-based host molecules and dicationic dyes offer new and improved characteristics making them particularly suitable for the detection of polyamines, in particular for the detection of biogenic polyamines.

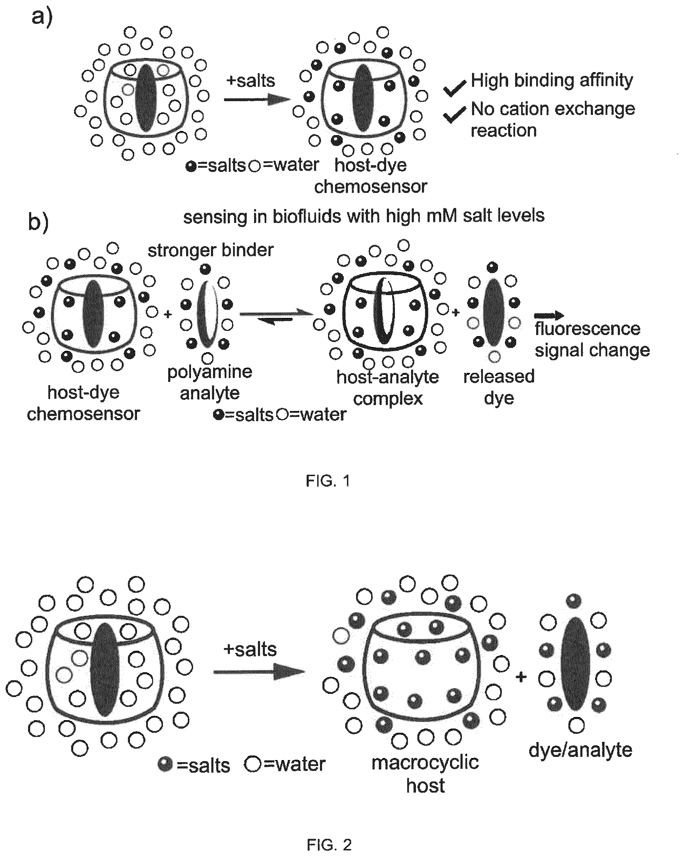

Herein, the term "chemosensor(s)" in the sense of the invention refers to the host molecule as described herein loaded with one or more indicator dyes. For the preparation of the chemosensors described therein, the indicator dye molecules are introduced (loaded) individually into the host molecules, which act as molecular containers, and are reversibly deposited therein or conjugated with them. By displacement of the dye from the host molecule by the analytes (polyamines) the indicator dye is reversibly released. Reversibly dye-loaded host molecules and the reversible dye loading and displacement are illustrated in Figure 1.

The inventors found that the specific selection of host molecule (receptor) and indicator dyes, as described herein, allows for achieving both a sensitive and selective method for polyamine detection. In the chemosensors of the invention, the pillar[n]arene-based host molecules act as molecular containers and, in combination with the selected indicator dyes, become suitable for polyamine detection also in an assay format. The system exhibits good functionality in biofluids, is stable even at very high salt concentrations (up to 1 M salt) and can be easily synthesised from cost-efficient starting materials. Therewith, analytical systems can be designed allowing to achieve a rapid response leading to a superior "turn-on" fluorescence signal in the sensing assay within seconds.

As explained above, the so far known fluorescent chemosensors are only suitable for use in media with low salt concentrations. At high salt concentrations such as present in biofluids or biologically relevant media, established systems suffer, for instance, from undesirable cation exchange reactions and interferences on the charge-mediated recognition process between the negatively charged chemosensor and the polycationic polyamines. Moreover, this can severely affect the stability of fluorescent chemosensors self-assembled from a negatively charged receptor and a cationic dye. As a result, the established systems become dysfunctional in biologically relevant media such as PBS, blood, urine, or saliva that contain high millimolar salt concentrations. This is illustrated in Figure 2.

In contrast, the new host-dye chemosensors, according to the invention, provide chemosensors wherein the dicationic dye exhibits a relatively strong binding affinity with the negatively charged receptor host molecule, which ensures the stability of the chemosensors at high salt concentrations, avoiding any cation exchange reactions (see Figure 1a). Moreover, the polycationic polyamines such as spermine, spermidine, and cadaverine also exhibit significantly strong binding affinities with the receptor host molecule, even at high salt concentrations. Sensing is achieved via the fluorescence signal change upon displacement of the dye from the host cavity by the stronger binding polyamines upon addition of the novel host-dye chemosensor into the polyamine-containing sample media (see Figure 1b). The strong binding affinity between the analyte and the receptor prohibits any salt interferences on the charge-mediated recognition event.

The new host-dye chemosensors, according to the invention, are self-assembled chemosensors. Further, the new chemosensors are optically active chemosensors. That means that the chemosensors are chemosensors with optical signal transduction capabilities.

The host molecule of the novel chemosensors, according to the invention, are selected from pillar[*n*]arene-based host molecules. Examples comprise anionic water-soluble pillar[n]arenes including anionic functional groups, such as carboxylates, sulfonates, sulphates, or phosphonates.

Preferably, the pillar[n]arene-based host molecule is selected from sulfated pillar[n]arenes. Examples comprise sulfated pillar[5]arene (P5AS), sulfated pillar[6]arene (P6AS), or sulfated pillar[7]arene (P7AS).

The most preferred host molecule is sulfated pillar[5]arene (P5AS), having the following structure:

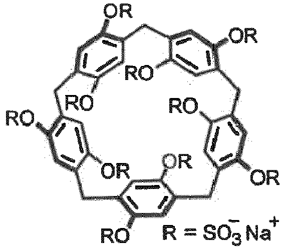

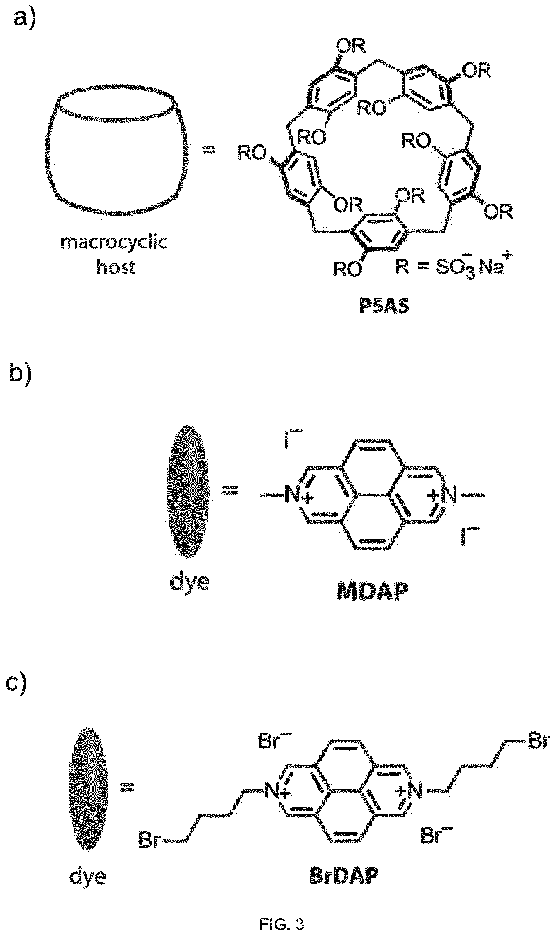

This macrocyclic host is also illustrated in Figure 3a.

Pillar[n]arene-based host molecules, such as particularly P5AS, show ultrahigh binding affinities towards guests containing quaternary ammonium ions due to the negative charge density at the portals and further display good aqueous solubility. The host molecule itself is non-chromophoric. For the preferred use in physiological or biologically relevant media, the host molecules should exhibit good aqueous solubility.

To obtain an optically active chemosensor, an indicator dye that equips the chemosensor with optical signal transduction capabilities is required. The dyes cause a signal change in the absorption, transmission, or fluorescence spectrum. For the preferred use in physiological or biologically relevant media, the indicator dye should exhibit good aqueous solubility. Further, the indicator dye should exhibit a strong binding affinity with the host molecule in order to prevent the dissociation of the complex at high salt concentrations.

The inventors found that fluorescent dyes, in particular, dicationic dyes having at least two quaternary ammonium groups, are suitable to obtain the chemosensor with optical signal transduction capabilities of the invention. Dicationic dyes should be selected which are "size-fitting", which means the dyes must be able to enter the cavities of the chemosensor, e.g., of P5AS. Further, the dyes should exhibit a binding affinity of *K*ₐ = 10⁵ M⁻¹ with M=mol/L or more. Examples of dicationic dyes having at least two quaternary ammonium groups comprise diazapyrenium-based dyes, diazaperoperylenium-based dyes, MDPP and BDAP methyl viologen, lucigenin, etc. Diazapyrenium-based dyes and diazaperoperylenium-based dyes are preferred.

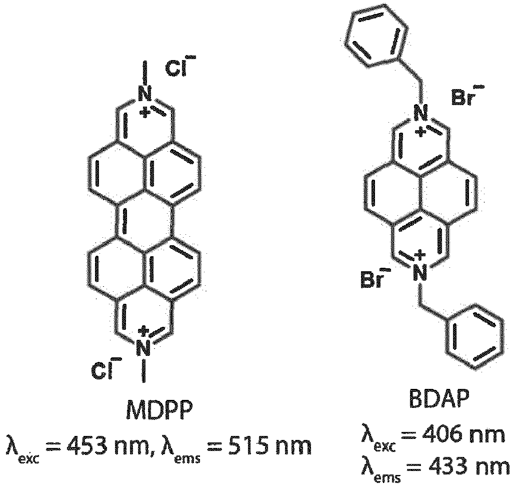

In particular, novel chemosensors with optical signal transduction capabilities contain a fluorescent dicationic dye.

With respect to fluorescent chemosensors, it should be noted that the fluorescent chemosensors reported to date have mostly exhibited an inferior fluorescence "turn-off' phenomenon accompanied by slow signal responses in polyamine sensing assays. This is undesirable when conducting regular monitoring of polyamine levels in biofluids and searching for concentration fluctuations in relation to the fluorescence signal change. Hence, the distinction between healthy and non-healthy patient samples is difficult to achieve. Moreover, a fast responding system is required for rapid and comprehensive analysis of biofluid samples.

Further, the previously reported fluorescent chemosensors for polyamines suffer from strong interferences with other biomolecules in the media, such as amino acids and other biogenic amines, proteins, and nucleobases, and hence lack selectivity for the polyamines of interest. There it is difficult to detect and distinguish polyamines in the media with these systems due to the lack of a selective signal readout. Moreover, the strong sample-to-sample matrix differences make the evaluation of the analytical assay almost impossible.

To provide improved host-dye fluorescent chemosensors, which do not suffer from the mentioned disadvantages, the inventors developed and evaluated chemosensors based on receptor hosts in combination with indicator dyes with the particular suitability to provide selective polyamine sensing also in biofluids. As explained above, the indicator dyes, according to the invention, exhibit relatively strong binding affinity towards the receptor host molecule. This binding affinity is also crucial in determining the selectivity of the sensor for the polyamines of interest. Among the analytes present in the biofluid media, the polycationic polyamines, in particular biogenic polyamines such as spermine, spermidine, and cadaverine, show the strongest binding affinities towards the receptor host molecule and hence can displace the dye from the host, while other weaker binding analytes are not able to displace the dye. The displacement of the dye leads to a selective fluorescence "turn-on" signal output.

The functionality of the chemosensor depends largely on the selected indicator dye.

For providing fluorescent chemosensors, the dicationic dye is particularly selected from dicationic dyes having a diazapyrenium core, such as *N*,*N*'-dimethyl-2,7-diazapyrenium dication (MDAP), *N*,*N*'-bis(4-bromobutyl)-2,7-diazapyrenium dication (BrDAP), *N*,*N*'-dimethyl-4,9-diazapyrenium dication, *N*,*N*'-bis(2-propenyl)-2,7-diazapyrenium dication, etc.

Most preferred are dicationic dyes selected from MDAP and BrDAP according to the following structures:

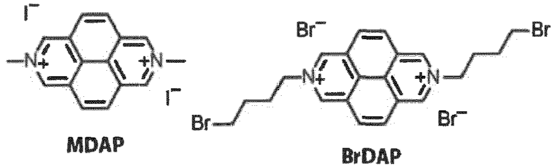

The dicationic diazapyrenium-based indicator dye MDAP (illustrated in Figure 3b) turned out to be highly emissive. The dicationic diazapyrenium dye derivative BrDAP (illustrated in Figure 3c), having alkyl side chains, exhibits similar properties as MDAP and has a 10² times stronger binding affinity for P5AS compared to MDAP.

Further examples of dicationic dyes include diazapyrenium-based dyes, diazaperoperylenium-based dyes, methyl viologen, lucigenin, etc., among which diazapyrenium-based dyes and diazaperoperylenium-based dyes are preferred.

As explained herein and as shown in the example part further below, the inventors achieved the desired results of combined polyamine detection and selective detection of target polyamines with diazapyrenium-based dyes, in particular as shown here with MDAP and BrDAP.

The chemosensors of the invention can be obtained as self-assembled chemosensors by mix-and-shake of host and dye solutions.

A preferred chemosensor of the invention relates to a fluorescent chemosensor comprising a sulfated pillar[n]arene (P5AS) having the structure as shown in Figure 3a and a dicationic dye selected from MDAP and/or BrDAP as shown in Figures 3b and 3c, hereinafter also designated as P5AS-MDAP host-dye fluorescent chemosensor and P5AS-BrDAP host-dye fluorescent chemosensor, respectively.

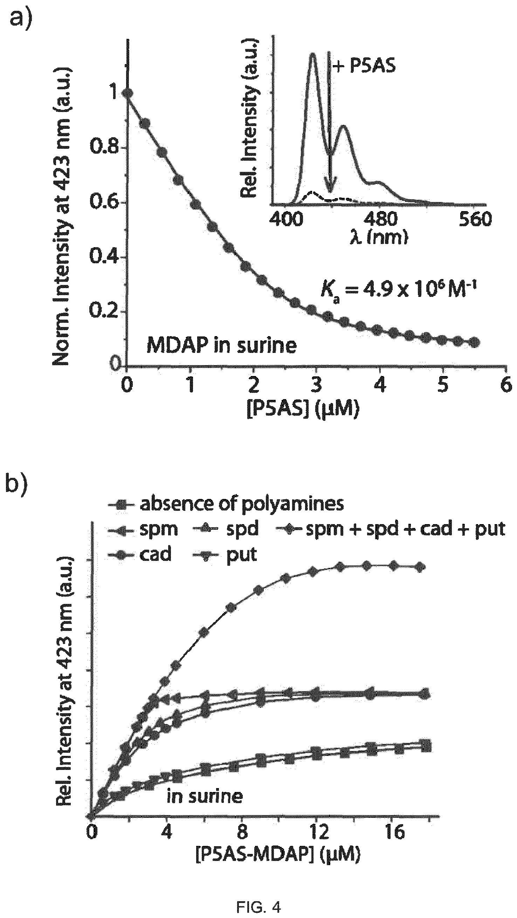

The inventors of the present invention were able to show that, for example, a mix-and-shake of the host and dye solutions results in the self-assembled P5AS-MDAP host-dye fluorescent chemosensor, for which the binding is instantaneous. Such self-assembled host-dye chemosensors turned out to be stable with relatively high binding affinities (*K*ₐ ~ 10⁶ M⁻¹) in both saline buffers (such as PBS) and biorelevant media, as can be derived from Figure 4a, showing experiments conducted in artificial urine (surine). The presence of spermine, spermidine, or cadaverine in the media leads to significant fluorescence changes in the chemosensor signal even in the presence of high salt content or biofluid-typical interferents, as shown in Figure 4b.

Addition of the receptor host molecule to the dye solution results in a quenching of the dye emission upon the formation of the self-assembled P5AS-MDAP host-dye fluorescent chemosensor. Thereby, in the sensing assay, in the presence of polyamines, the dye is displaced from the host cavity resulting in a fluorescence "turn-on" signal. The involved processes show very fast binding kinetics and fast signal responses.

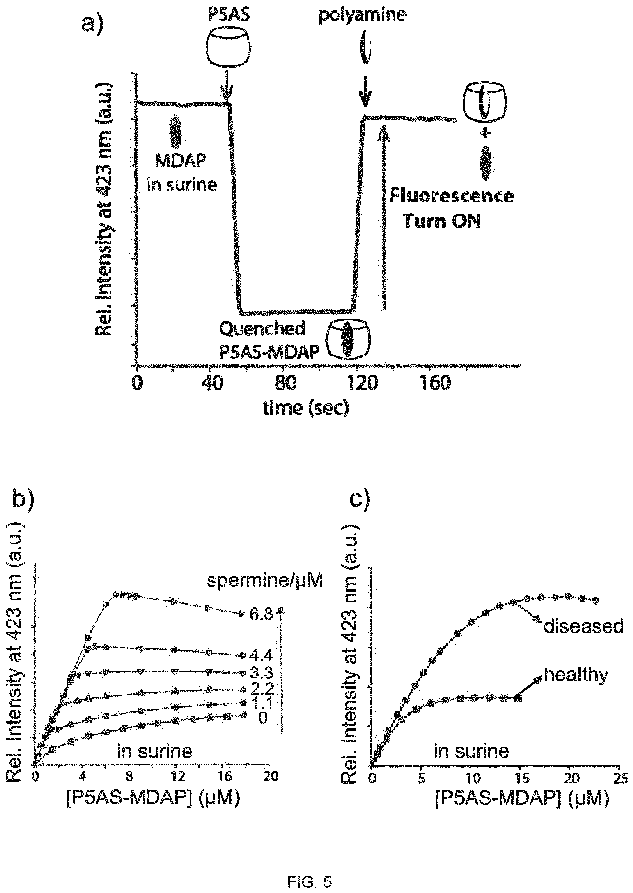

The new polyamine chemosensors further achieve a fluorescence "turn-on" signal as output. As can be derived from Figure 5a, the preferred indicator dye MDAP exhibits a strong fluorescence, which is strongly quenched upon the addition of the preferred P5AS host molecule resulting in the self-assembled chemosensor. The self-assembly and signal response is very fast and takes place within a few milliseconds (see Figure 5a). The addition of polyamines to the fluorescence-quenched chemosensor results in a displacement of the dye from the host by the stronger binding polyamines, and a fluorescence "turn-on" signal is achieved within milliseconds (see Figure 5a).

Moreover, it is possible to distinguish the different concentrations of spermine in surine upon the addition of the chemosensor to the samples through the fluorescence "turn-on" signal output. A 1 µM detection of spermine was achieved (Figure 5b). Furthermore, the addition of the chemosensor to artificial urine (surine) samples with a simulated concentration range of polyamine mixtures corresponding to the urine of healthy individuals and diseased patients with elevated polyamine levels resulted in distinctly distinguishable fluorescence "turn-on" signal outputs (see Figure 5c).

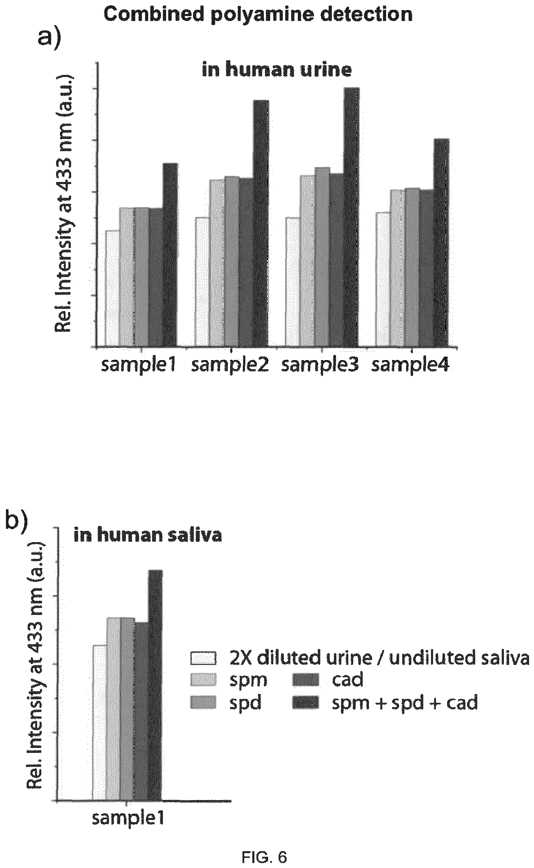

The sensing assay, as described in more detail in the following example section, was evaluated on a microplate reader in human urine and saliva samples collected from healthy individuals and later spiked with polyamines to simulate the urine/saliva from diseased patients. The sensing assay enables the selective detection of polyamines after the addition of chemosensors according to the invention using preferably P5AS-MDAP self-assembled chemosensor. A combined polyamine detection and differentiation between normal and elevated polyamine levels in biofluids can be achieved, which is advantageous for early disease detection as needed for cancer or Alzheimer's patients. The results are shown in Figure 6. This offers a promising advantageous approach for early disease detection, such as in cancer or Alzheimer's patients.

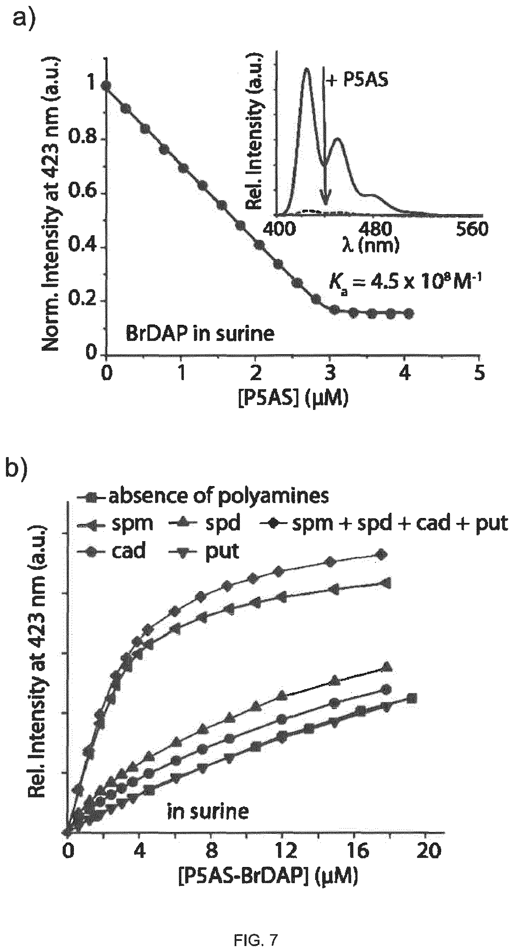

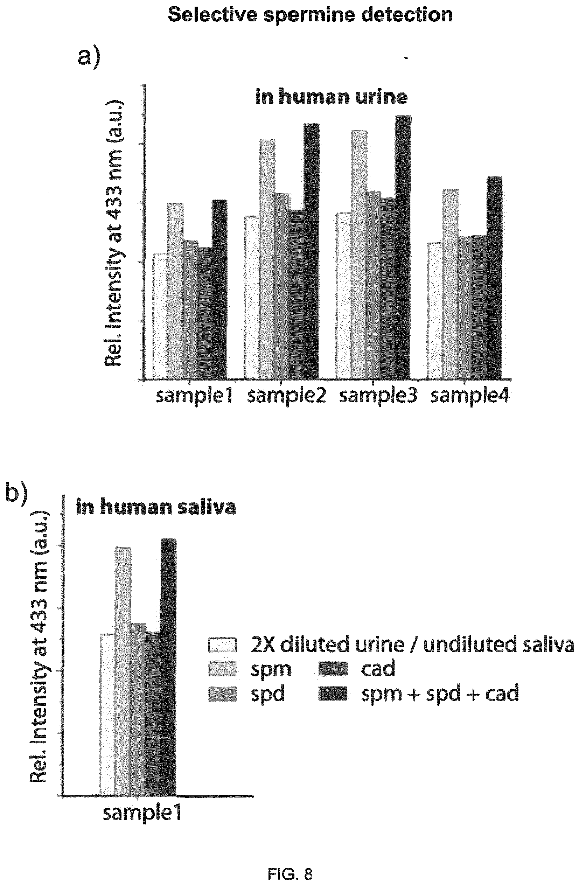

The further preferred self-assembled P5AS-BrDAP host-dye chemosensor as described above turned out to be selective only to spermine in the media, which is the strongest binder to the host amongst the evaluated polyamines. BrDAP displays a binding affinity of 10⁸ M⁻¹ towards P5AS. Figure 7a shows the results of experiments conducted in artificial urine (surine). In contrast to the P5AS-MDAP host-dye chemosensor, other polyamines such as spermidine and cadaverine cannot displace the BrDAP dye from the host in the P5AS-BrDAP host-dye chemosensor, which results in no significant fluorescence signal change (see Figure 7b). The spermine selectivity of the chemosensor evaluated in human urine and saliva samples collected from healthy individuals and later spiked with polyamines to simulate the urine/saliva from diseased patients shows the selective detection of spermine upon the addition of the P5AS-BrDAP chemosensor. Only samples spiked with spermine resulted in distinguishable fluorescence signal outputs from the healthy samples (see Figure 8).

Chemosensors selective for individual polyamines are important to monitor their independent physiological effects in the body and to devise personalised medicine strategies. Careful selection of host molecule and dye among those described herein offers various options to provide optimised chemosensors for the desired needs and relevant target analytics.

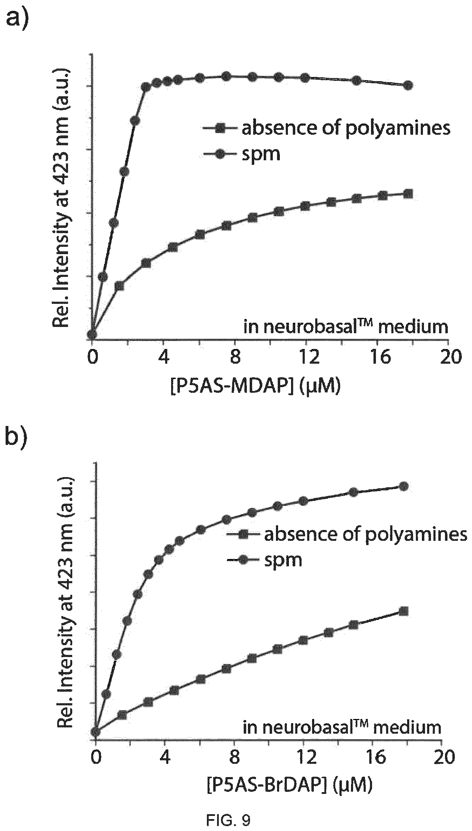

The chemosensors of the present invention are particularly suitable for the detection of polyamines in physiological media or endogenous fluids or secretions, including, for example, PBS, urine, saliva, blood, sweat, semen, amniotic fluid, tear fluid, cerebrospinal fluid, etc. In particular, the selectivity of the two preferred chemosensors for polyamines, P5AS-MDAP and P5AS-BrDAP, is further depicted by testing the sensing assay in more complex biorelevant media, such as neurobasal^{™} medium, which contains 37 different components, including amino acids, vitamins, and inorganic salts. The addition of the chemosensors to neurobasal^{™} medium containing spermine results in a strong fluorescence "turn-on" signal upon using both MDAP and BrDAP as indicator dyes (see Figure 9). This further confirms that the novel chemosensors, according to the invention, do not suffer from any significant matrix interferences and selectively detect the polyamines of interest.

Accordingly, a further aspect of the invention relates to the use of the new chemosensors for detecting and determining polyamines, in particular biogenic polyamines. Preferably, the polyamines are selected from polycationic polyamines. More preferably, the polyamines are selected from the group comprising spermine, spermidine, and cadaverine.

More particularly, this aspect of the invention relates to the use of the new chemosensors for detecting and determining biogenic polyamines in a fluorescence assay.

As explained above, one particular advantage of the present invention is the suitability for using the new chemosensors for detecting and determining biogenic polyamines in saline or physiological media or biologically (relevant) media. In the sense of the present invention, saline or physiological media, or biologically (relevant) media include, for example, buffers, such as PBS, or physiological saline but also biological (relevant) media such as artificial CSF or endogenous fluids, and secretions such as urine, digestive secretions, such as saliva, gastric juice, pancreatic secretions, or bile, blood, lymphatic fluid, cerebrospinal fluid, semen, amniotic fluid, lacrimal fluid, tear fluid, or sweat. Preferably, the aqueous medium is selected from the group comprising water, physiological saline, PBS, artificial CSF, urine, saliva, blood (comprising blood serum/human serum (HS) and human serum albumin (HSA)), CSF, amniotic fluid, semen, and sweat. Preferably, the aqueous medium is selected from the group comprising water, PBS, urine, saliva, blood, and CSF. Most preferably, the aqueous medium is selected from the group comprising water, PBS, urine, saliva, and blood. Also, artificial body fluids like artificial urine, also called "surine", or artificial biofluids, such as the so-called "neurobasal^{™} medium", are included herein.

Body fluids preferably relate to mammal body fluids, including human and animal body fluids.

Accordingly, a preferred aspect of the invention relates to the use of the new chemosensors for detecting and determining biogenic polyamines in physiological media or endogenous fluids or secretions, preferably in PBS, urine, saliva, blood, sweat, semen, amniotic fluid, tear fluid, or cerebrospinal fluid.

The invention further relates to a method for preparing the chemosensors as described herein by mixing an aqueous solution containing one or more host molecules described herein with an aqueous solution containing one or more of the dicationic dyes as described herein and shaking the mixture to obtain a self-assembled host-dye chemosensor.

In principle, it is also possible to prepare mixed self-assembled host-dye chemosensors by mixing an aqueous solution containing different host molecules with an aqueous solution containing different dicationic dyes as described herein (or to load one kind of host molecule with different dyes or load different host molecules with one kind of indicator dye) and shaking the mixture to obtain mixed self-assembled host-dye chemosensors.

A further aspect of the invention relates to a method for detecting, determining, or analysing polyamines with fluorescence spectroscopy by using the chemosensors as described herein. Determination and analysis of polyamines comprise identification and/or qualitative and quantitative evaluation of the polyamines.

The method for detecting, determining, or analysing polyamines comprises, in particular, the following steps:
i) preparing or providing the chemosensors as described herein;
ii) providing the chemosensors of step i) in an aqueous solution;
iii) providing an aqueous test sample;
iv) contacting the chemosensors of step i) or ii) with the test sample iii);
v) determination of the polyamines by fluorescence spectroscopy.

In one aspect of the invention, the method for detecting, determining, or analysing polyamines comprises the following steps:
i) preparing or providing the chemosensors as described herein, preferably by preparing the chemosensors with the method described above, e.g., by mix-and-shake methods;
ii) providing the chemosensors of step i) in an aqueous solution;
iii) mixing the aqueous solution of the chemosensors of step i) or ii) with an aqueous test sample;
iv) determination of the polyamines in the solution according to iii) by fluorescence spectroscopy.

The method as described herein may comprise an additional step of preparing a layer
(a) of the aqueous solution according to ii), or
(b) of a mixture of the chemosensors according to i) and the aqueous test sample according to iii), or
(c) of a mixture of the aqueous solution according to ii) and the aqueous test sample according to iii)
by means of a spraying or aerosol printing method on a support material and determination of polyamines in such a layer by fluorescence spectroscopy.

That means it is possible to transfer the aqueous solution ii), comprising the chemosensor i), with or without the test sample iii) into a layer format. It is also possible to transfer the chemosensor i) in a mixture with the test sample iii) into a layer format. If the layer is formed without the test sample iii), then the chemosensor layer must be contacted with the test sample iii) prior to the polyamine determination step.

The above-mentioned optional step of forming a layer relates to a further embodiment of the invention, wherein an aqueous solution comprising the chemosensors is converted into a layer, in the sense of a film or coating, by means of spray printing or aerosol printing processes. By producing a so-called "ink", the aqueous chemosensor solution can be applied in several ultrathin layers to various surfaces of a carrier, e.g., of glass, paper, or plastic, such as glass slides, quartz, or PS surfaces, by means of spray printing processes and anchored to the surface of the carrier by the evaporation of the liquid components of the aerosol. Such layers, films, or coatings from the aqueous chemosensor solution are also suitable for fluorescence determination methods.

In the above-described determination methods, the polyamines are preferably selected from polycationic polyamines, more preferably the polycationic polyamines are biogenic polyamines, such as preferably selected from the group comprising spermine, spermidine, and cadaverine.

In the above-described methods, the test sample, e.g., of step iii), is preferably a biologically relevant fluid, such as an endogenous fluid or secretion sample obtained from a human or an animal, such as urine, saliva, blood, sweat, semen, amniotic fluid, tear fluid, or cerebrospinal fluid.

When using the methods described above for detecting, determining, or analysing spermine, the chemosensor preferably comprises BrDAP as the dicationic diazapyrenium dye.

The inventors of the invention found that the compounds MDAP and BrDAP, as shown in Figures 3b and 3c are suitable to act as a fluorescence dye in an assay for detecting and determining polyamines, in particular biogenic polyamines, more particularly polycationic polyamines, such as preferably spermine, spermidine and cadaverine. Therefore, a further aspect of the invention relates to the use of MDAP and BrDAP as fluorescence dyes in respective assays.

The invention further relates to diagnostic kits (kit-of-parts), which include a chemosensor comprised of a host molecule as described herein and a dye molecule as described herein.

Such diagnostic test kits offer a comfortable approach for broadly applicable analysis of polyamines, in particular biogenic polyamines, more particularly polycationic polyamines, such as preferably spermine, spermidine, and cadaverine. Such kits may be designed as ready-to-use test kits for laboratory use or alternatively may be in the form of ready-to-use kits for easy-to-apply tests directly at home or in doctor's offices etc. Further examples include medical laboratory diagnostics and home diagnostics with printable sensor chips, bench-top applications with rapid tests, and "lifestyle products" for customers interested in their spermidine levels, which is a known "geroprotector". In particular, such diagnostic tests are designed for testing human (or animal) urine samples.

The invention can be used in the field of analytical chemistry and physiological analysis, including medical diagnostics, early disease detection, personalized medicine strategies, life science research, and instrumental analysis.

In summary, the present invention provides the following advantages:
➢ use of new self-assembled host-dye fluorescent chemosensors with strong binding affinity in saline buffers and biorelevant media;
➢ high stability of the system, avoiding any cation exchange reactions;
➢ host and indicator dye molecules can easily be prepared from cost-efficient starting materials;
➢ the chemosensors, host molecules, and indicator dyes exhibit good aqueous solubility;
➢ polyamine detection tests in saline buffers and biorelevant media such as surine using the new chemosensors display good functionality and are not interfered by salt presence in the sensing assay;
➢ addition of chemosensors to sample media with polyamines results in a fluorescence "turn-on" signal, which assists in differentiating the concentration fluctuations of polyamines in biofluids in relation to the fluorescence signal variations with significant sensitivity;
➢ the new chemosensors allow for achieving a combined polyamine detection (spermine + spermidine + cadaverine);
➢ selectivity of the new chemosensors for polyamines of interest demonstrated through measurements in human urine and saliva, showing clearly distinguishable fluorescence signals observed for samples collected from healthy individuals and later spiked with polyamines to simulate the concentrations in diseased patients;
➢ selectivity of the chemosensors for polyamines and no interferences with other biomolecules in the media confirmed by the determination of polyamines in complex solutions such as neurobasal^{™} medium.

### DESCRIPTION OF THE FIGURES

- Fig. 1: Schematic illustration of cation exchange reaction in systems according to the invention, wherein
(a) illustrates the strong binding affinity between the dye and the host in the self-assembled host-dye chemosensor, which prohibits any cation exchange reactions at high salt concentrations, so that the dye remains intact inside the host, ensuring the stability of the chemosensor; and wherein
(b) illustrates the sensing assay, wherein the addition of the self-assembled host-dye chemosensor of the invention into biofluids containing polyamines results in the displacement of the dye from the host by the stronger binding polyamine accompanied by a fluorescence signal change.
- Fig. 2: Schematic illustration of established cation exchange reactions in systems wherein the known chemosensors become dysfunctional in biologically relevant media such as PBS or urine. At high salt concentrations, the signalling binding pockets of the receptor host molecule are occupied by salts and are no longer available for the detection of relevant analytes. Moreover, chemosensors self-assembled from a receptor host and dye molecules decompose at high salt concentrations as the dye is pushed out of the receptor by the salts.
- Fig. 3: (a) Chemical structure of the preferred host molecule P5AS.
(b) Chemical structure of the preferred dye molecule MDAP.
(c) Chemical structure of the preferred dye molecule BrDAP.
- Fig. 4: (a) Normalised fluorescence intensity monitored at 423 nm of the preferred indicator dye MDAP (2.34 µM) in surine upon stepwise addition of the preferred host molecule P5AS, resulting in a quenching of the dye fluorescence. The host-dye binding interaction in surine shows a binding affinity (*K*ₐ) of 4.9 × 10⁶ M⁻¹. The inset shows the fluorescence spectra of MDAP (2.34 µM) in surine before (solid line) and after (dashed line) addition of P5AS (5.5 µM).
(b) The relative fluorescence intensity changes monitored at 423 nm upon addition of the preferred P5AS-MDAP host-dye chemosensor to a solution of spermine (spm, 3.3 µM), spermidine (spd, 3.3 µM), cadaverine (cad, 3.3 µM), putrescine (put, 3.3 µM), and a mixture of all four polyamines (each at 3.3 µM) in surine. The strong fluorescence enhancement observed in the case of spermine, spermidine, and cadaverine displays the functionality of the sensor for polyamine detection. Putrescine does not give rise to any changes in the fluorescence intensity and hence does not interfere with the sensing assay.
- Fig. 5: (a) Relative fluorescence intensity changes with time monitored at 423 nm of MDAP (3.1 µM) in surine upon addition of P5AS (3.1 µM), resulting in the instantaneous formation of the fluorescence-quenched P5AS-MDAP self-assembled chemosensor within a few milliseconds. The addition of the stronger binding spermine (5.1 µM) to the media results in the displacement of MDAP from the host and a fluorescence signal "turn-on" is achieved within a few milliseconds.
(b) The relative fluorescence intensity changes monitored at 423 nm upon addition of P5AS-MDAP chemosensor to a solution of surine with varying concentrations of spermine (0 - 6.8 µM) resulting in distinguishable fluorescence "turn-on" signal readouts.
(c) Relative fluorescence intensity changes monitored at 423 nm on addition of P5AS-MDAP chemosensor to surine samples with simulated concentrations of polyamines corresponding to urine of healthy individuals (1.1 µM spm + 1.3 µM spd + 2.1 µM cad + 1.9 µM put) and diseased patients (5.8 µM spm + 3.2 µM spd + 7.8 µM cad + 7.4 µM put); (spermine = spm, spermidine = spd, cadaverine = cad, and putrescine = put).
- Fig. 6: Relative fluorescence intensity changes monitored at 433 nm on addition of the preferred P5AS-MDAP chemosensor (21.6 µM) according to the invention to
(a) human urine samples collected from four healthy individuals (diluted two times) and later spiked with several polyamines and polyamine mixtures (each at 5 µM) in order to simulate urine from diseased patients and
(b) human saliva samples collected from a healthy individual (undiluted) and later spiked with several polyamines and polyamine mixtures (each at 5 µM) in order to simulate saliva from diseased patients (microplate reader assay) showing a clear distinction between the fluorescence signal outputs from healthy biofluid samples and polyamine-spiked samples in all cases; (spermine = spm, spermidine = spd, cadaverine = cad and putrescine = put).
- Fig. 7: (a) Normalised fluorescence intensity monitored at 423 nm of the preferred indicator dye BrDAP (2.95 µM) in surine upon stepwise addition of the preferred host molecule P5AS, resulting in a quenching of the dye fluorescence. The host-dye binding interaction in surine shows a binding affinity (*K*ₐ) of 4.5 × 10⁸ M⁻¹. The inset shows the fluorescence spectra of BrDAP (2.95 µM) in surine before (solid line) and after (dashed line) addition of P5AS (4.4 µM).
(b) The relative fluorescence intensity changes monitored at 423 nm upon addition of the preferred P5AS-BrDAP host-dye chemosensor to a solution of spermine (spm, 3.3 µM), spermidine (spd, 3.3 µM), cadaverine (cad, 3.3 µM), putrescine (put, 3.3 µM), and a mixture of all polyamines (each at 3.3 µM) in surine. The strong fluorescence enhancement observed only in the case of spermine indicates the selectivity of the chemosensor for spermine detection.
- Fig. 8: Relative fluorescence intensity changes monitored at 433 nm on addition of P5AS-BrDAP chemosensor (21.6 µM) to
(a) human urine samples collected from four healthy individuals (diluted two times) and later spiked with several polyamines and polyamine mixtures (each at 5 µM) in order to simulate urine from diseased patients and
(b) human saliva sample collected from a healthy individual (undiluted) and later spiked with several polyamines and polyamine mixtures (each at 5 µM) in order to simulate saliva from diseased patients (microplate reader assay). A clear distinction between the fluorescence signal outputs is observed only from healthy urine samples and urine samples spiked with spermine in all cases indicating the selectivity of the chemosensor for spermine; (spermine = spm, spermidine = spd, cadaverine = cad and putrescine = put).
- Fig. 9: Relative fluorescence intensity changes monitored at 423 nm upon addition of
(a) P5AS-MDAP host-dye chemosensor and
(b) P5AS-BrDAP host-dye chemosensor to a solution of spermine (spm, 3.3 µM) in neurobasal^{™} medium. A strong fluorescence enhancement observed in the presence of spermine in this complex media indicates the selectivity of both chemosensors for polyamine detection without any matrix interferences.

### EXAMPLES

The invention is described further by the following examples, but without being limited thereto.

### Example 1 - Preparation of a P5AS-MDAP host-dye chemosensor

The P5AS-MDAP host-dye chemosensor, according to the invention, has been prepared as follows:
- A stock solution of the host P5AS was prepared in PBS at a concentration of 2 mM in an Eppendorf tube.
- A stock solution of the indicator dye MDAP was prepared in PBS at a concentration of 2 mM in an Eppendorf tube.
- A 100 µM P5AS-MDAP host-dye chemosensor solution suitable for utilization in the polyamine sensing assay was prepared by mixing 280 µL of the P5AS stock solution and 280 µL of the MDAP stock solution in 5000 µL PBS at a 1: 1 ratio in an Eppendorf tube. The solution was then mixed by shaking for 30 seconds, which resulted in the instantaneous formation of the P5AS-MDAP chemosensor at a concentration of 100 µM.
- The P5AS-MDAP host-dye chemosensor solution was freshly prepared from their respective host and dye stock solutions each time before the start of the polyamine sensing assay.

### Example 2 - Preparation of a P5AS-BrDAP host-dye chemosensor

The P5AS-BrDAP host-dye chemosensor, according to the invention, has been prepared as follows:
- A stock solution of the host P5AS was prepared in PBS at a concentration of 2 mM in an Eppendorf tube.
- A stock solution of the indicator dye BrDAP was prepared in PBS at a concentration of 2 mM in an Eppendorf tube.
- A 100 µM P5AS-BrDAP host-dye chemosensor solution suitable for utilization in the polyamine sensing assay was prepared by mixing 280 µL of the P5AS stock solution and 280 µL of the BrDAP stock solution in 5000 µL PBS at a 1: 1 ratio in an Eppendorf tube. The solution was then mixed by shaking for 30 seconds, which resulted in the instantaneous formation of the P5AS-BrDAP chemosensor at a concentration of 100 µM.
- The P5AS-BrDAP host-dye chemosensor solution was freshly prepared from their respective host and dye stock solutions each time before the start of the polyamine sensing assay.

### Example 3 - Preparation of further P5AS-dye chemosensors

Further host-dye chemosensors have been prepared as follows, using P5AS as the host molecule and, e.g., MDPP or BDAP as indicator dyes.
- A stock solution of the host P5AS was prepared in PBS at a concentration of 2 mM in an Eppendorf tube.
- A stock solution of the indicator dye was prepared in PBS at a concentration of 2 mM in an Eppendorf tube.
- A 100 µM P5AS-dye chemosensor solution suitable for utilization in the polyamine sensing assay was prepared by mixing 280 µL of the P5AS stock solution and 280 µL of the indicator dye stock solution in 5000 µL PBS at a 1: 1 ratio in an Eppendorf tube. The solution was then mixed by shaking for 30 seconds, which resulted in the instantaneous formation of the P5AS-dye chemosensor at a concentration of 100 µM.
- The P5AS-dye chemosensor solution was freshly prepared from their respective host and dye stock solutions before the start of the polyamine sensing assay.

### Example 4 - Determination of polyamines using P5AS-MDAP host-dye chemosensor

A fluorescence assay for the combined polyamine detection has been carried out using the P5AS-MDAP host-dye chemosensor according to the invention as follows:
- The polyamine sensing assay (combined polyamine detection) was conducted using a microplate reader capable of fluorescence detection by setting the excitation wavelength at 406 nm and emission wavelength at 433 nm.
- The different samples for analysis, for, e.g., human urine or saliva samples, were collected from healthy individuals and also later spiked with polyamines, in particular, spermine, spermidine, and cadaverine, in order to simulate the urine/saliva samples from diseased patients.
- 200 µL of the test samples were filled into the wells of a black opaque 96-well microplate for the measurement.
- The background fluorescence intensity was initially monitored and recorded at the microplate reader from the samples alone prior to the addition of the P5AS-MDAP host-dye chemosensor.
- Following this, 55 µL of the P5AS-MDAP host-dye chemosensor stock solution (at a concentration of 100 µM) was titrated into the samples in the microplate wells to arrive at a concentration of 21.6 µM chemosensor in the wells. The resultant solution was mixed for 30 seconds by using the plate shaking feature of the microplate reader.
- The fluorescence intensity was monitored and recorded after the addition of the P5AS-MDAP host-dye chemosensor to the sample. The background fluorescence intensity from the samples alone was subtracted from the observed fluorescence intensity after the addition of the chemosensor for each well to arrive at the resultant fluorescence "turn-on" signal response.
- The combined polyamine levels in the samples were determined from their respective fluorescence "turn-on" signal responses.

### Example 5 - Determination of polyamines using P5AS-BrDAP host-dye chemosensor

A fluorescence assay for the selective spermine detection has been carried out using the P5AS-BrDAP host-dye chemosensor according to the invention as follows:
- The polyamine sensing assay (selective spermine detection) was conducted using a microplate reader capable of fluorescence detection by setting the excitation wavelength at 406 nm and emission wavelength at 433 nm.
- The different samples for analysis, e.g., human urine or saliva samples, were collected from healthy individuals and also later spiked with polyamines, in particular, spermine, spermidine, and cadaverine, in order to simulate the urine/saliva samples from disease patients.
- 200 µL of the test samples were filled into the wells of a black opaque 96-well microplate for the measurement.
- The background fluorescence intensity was initially monitored and recorded at the microplate reader from the samples alone prior to the addition of the P5AS-BrDAP host-dye chemosensor.
- Following this, 55 µL of the P5AS-BrDAP host-dye chemosensor stock solution (at a concentration of 100 µM) was titrated into the samples in the microplate wells to arrive at a concentration of 21.6 µM chemosensor in the wells. The resultant solution was mixed for 30 seconds by using the plate shaking feature of the microplate reader.
- The fluorescence intensity was monitored and recorded after the addition of the P5AS-BrDAP host-dye chemosensor to the sample. The background fluorescence intensity from the samples alone was subtracted from the observed fluorescence intensity after the addition of the chemosensor for each well to arrive at the resultant fluorescence "turn-on" signal response.
- The spermine levels in the samples were determined from their respective fluorescence "turn-on" signal responses.

## Claims

1. A chemosensor comprising a pillar[n]arene-based host molecule and a dicationic dye.

2. The chemosensor according to claim 1, which is an optically active chemosensor containing a fluorescent dicationic dye.

3. The chemosensor according to any one of the claims 1 or 2, wherein the host molecule is a sulfated pillar[n]arene.

4. The chemosensor according to any one of the claims 1 to 3, wherein the host molecule is a sulfated pillar[n]arene (P5AS) having the structure

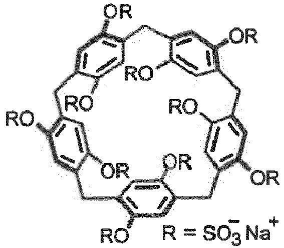

5. The chemosensor according to any one of the preceding claims, wherein the dicationic dye has at least two quaternary ammonium groups.

6. The chemosensor, according to any one of the preceding claims, wherein the dicationic dye has a diazapyrenium core.

7. The chemosensor, according to any one of the preceding claims, wherein the dicationic dye is selected from MDAP and BrDAP according to the following structures

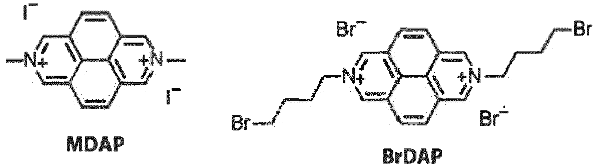

8. The use of the chemosensors according to any one of the claims 1 to 7 for detecting and determining polyamines, in particular biogenic polyamines, such as preferably polycationic polyamines.

9. The use according to claim 8, wherein the polyamines are selected from the group comprising spermine, spermidine, and cadaverine.

10. The use according to any one of the claims 8 or 9 for detecting and determining biogenic polyamines in a fluorescence assay.

11. The use according to any one of the claims 8 to 10 for detecting and determining biogenic polyamines in physiological media or endogenous fluids or secretions, preferably in PBS, urine, saliva, blood, sweat, semen, amniotic fluid, tear fluid, or cerebrospinal fluid.

12. A method for preparing the chemosensors according to any one of the claims 1 to 7 by mixing an aqueous solution containing the host molecules as defined in claims 1 to 5 with an aqueous solution containing the dicationic dye as defined in claims 1 or 6 to 8 and shaking the mixture to obtain a self-assembled host-dye chemosensor.

13. A method for detecting, determining, or analysing polyamines with fluorescence spectroscopy, by using the chemosensors according to any one of the claims 1 to 7, preferably for detecting, determining, or analysing polycationic polyamines, such as biogenic polyamines, preferably selected from the group comprising spermine, spermidine, and cadaverine.

14. The method according to claim 13, comprises the steps:
i) preparing or providing the chemosensors according to any of the claims 1 to 7;
ii) providing the chemosensors of step i) in an aqueous solution;
iii) providing an aqueous test sample, preferably a physiological medium such as PBS, an endogenous fluid or secretion sample obtained from a human or an animal such as urine, saliva, blood, sweat, semen, amniotic fluid, tear fluid, or cerebrospinal fluid;
iv) contacting the chemosensors of step i) or ii) with the test sample iii), wherein the chemosensors can be present either in aqueous solution or in the form of a layer prepared by means of a spraying or aerosol printing method on a support material;
v) determination of the polyamines by fluorescence spectroscopy.

15. A diagnostic kit comprising a chemosensor according to any one of claims 1 to 7.
